# DEMANDE DE BREVET EUROPEEN

(11) **EP 2 065 064 A1**
(43) Date de publication de la demande: **03.06.2009**
(21) Numéro de dépôt: 07121838.2
(22) Date de dépôt: 29.11.2007
(51) Int. Cl.: A61M 5/315, G01D 5/14, A61M 5/31

(54) **Stylo injecteur de medicament**

(71) Demandeur: Valtronic Technologies (Suisse) SA, 1343 Les Charbonnières (CH)
(72) Inventeur: Mauron, Frédéric, 1441, Valeyres Montagny (CH); Negrea, Dan, 1342, Le Pont (CH); Rochat, Michel, 1167, Lussy-sur-Morges (CH)
(74) Mandataire: GLN

(57) **Abrégé**

L'invention concerne un stylo injecteur de médicament liquide constitué d'une seringue comportant :
- un corps de pompe (10) destiné à contenir le médicament à injecter et muni d'une aiguille (11) ou d'une cartouche de liquide pré remplie,
- un piston (12) capable de coulisser dans le corps de pompe (10),
- une tige (13) à une extrémité de laquelle est fixé le piston (12),
- un fourreau (14) solidaire de la tige (13), capable de coulisser autour du corps de pompe (10),
- un boîtier (15) disposé à l'intérieur du fourreau (14) et capable d'être positionné en divers endroits de la tige (13) de manière à ajuster la course du piston (12),
- des moyens de mesure inductive linéaire (18, 20) de la course à effectuer par le piston (12),
- des moyens de détermination (21) de la quantité de médicament injectable qui correspond à la course mesurée du piston (12), et
- des moyens d'affichage (22) de ladite quantité.

## Description

### Domaine technique

La présente invention se rapporte au domaine de l'administration de médicaments. Elle concerne, plus particulièrement, un stylo injecteur de médicament liquide à affichage électronique de la dose à injecter.

### Etat de la technique

De tels stylos permettent au patient de procéder lui-même, avec facilité, sécurité et précision, à l'injection de son médicament, de l'insuline, par exemple. II existe pour cela différents types de stylos injecteurs qui peuvent avoir des systèmes d'affichage mécanique ou électronique.

II a été proposé des stylos électroniques intégrant un système de mesure électro-mécanique rotatif dans le but d'avoir un affichage électronique permettant d'indiquer les doses avec des caractères de plus grande dimension que dans des applications mécaniques et ainsi d'en faciliter la lecture. Le comptage des doses se fait soit à l'aide d'une came qui actionne des poussoirs lors de sa rotation soit de balais en contact avec des bagues conductrices. Dans le cas des poussoirs, chaque actionnement est comptabilisé et correspond à un volume unitaire qui sera injecté. Cependant, la mécanique liée aux poussoirs ou aux balais est complexe et nécessite un nombre élevé de composants, induisant un coût relativement élevé qu'il est important de réduire dans ce type d'applications thérapeutiques. De plus, la complexité de ces assemblages, basés sur des systèmes rotatifs, pénalise la fiabilité des systèmes d'injection.

Afin de s'affranchir de systèmes de mesure des doses basés sur des éléments rotatifs, le document US 6,068,615 propose une seringue jetable comprenant des matériaux magnétiques disposés sur son piston. Avant l'injection, la seringue est placée dans un support extérieur en forme de cylindre afin de mesurer de manière inductive et enregistrer la quantité de liquide présent, qui sera administrée au patient. Le recours à un équipement extérieur rend cette solution peu pratique, surtout en cas de déplacements fréquents du patient. De plus, celui-ci ne connaît la mesure qu'après avoir placé la seringue dans le cylindre. Enfin, il ne dispose d'aucune flexibilité sur la quantité de liquide à injecter et doit obligatoirement s'injecter l'intégralité du médicament présent dans la seringue.

### Divulgation de l'invention

Un objet de la présente invention est de fournir un stylo injecteur facile d'utilisation et peu encombrant pour le patient.

Un autre objet de l'invention est l'utilisation d'un système de mesure inductif, donc sans contact mécanique entre les éléments mobiles du stylo injecteur et les éléments fixes. Ce principe renforce la robustesse du système et de fait, en améliore sa fiabilité dans des conditions d'usage variables.

Un autre objet de la présente invention est de fournir un stylo injecteur permettant d'obtenir aisément, sans recours à un équipement extérieur, une indication de la dose à injecter, de la dose injectée et de la dose restante à disposition dans la seringue.

Pour atteindre tout ou partie de ces objets ainsi que d'autres, le stylo injecteur selon l'invention est constitué d'une seringue comportant:
- un corps de pompe destiné à contenir le médicament à injecter et muni d'une aiguille,
- un piston capable de coulisser dans le corps de pompe,
- une tige à une extrémité de laquelle est fixé le piston,
- un fourreau capable de coulisser autour du corps de pompe,
- un premier boîtier disposé à l'intérieur du fourreau et capable d'être positionné en divers endroits de la tige de manière à ajuster la course du piston,
- des moyens de mesure inductive de la course à effectuer par le piston,
- des moyens de détermination de la quantité de médicament injectable qui correspond à la course mesurée du piston, et
- des moyens d'affichage de ladite quantité.

D'autres caractéristiques de la présente invention apparaîtront plus clairement à la lecture de la description qui va suivre, faite en référence au dessin annexé, dans lequel:

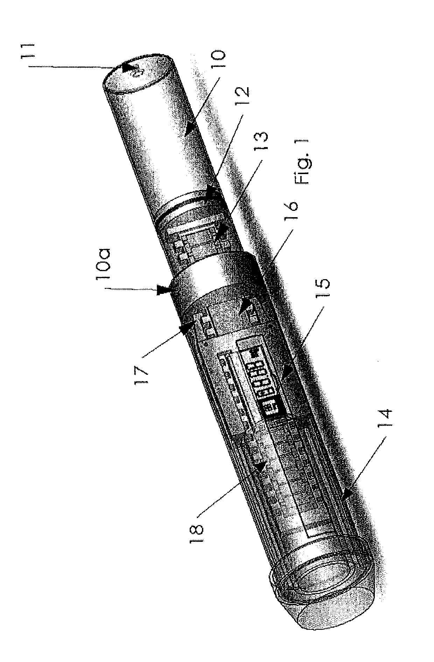

- la figure 1 est une vue générale d'un stylo injecteur selon l'invention;

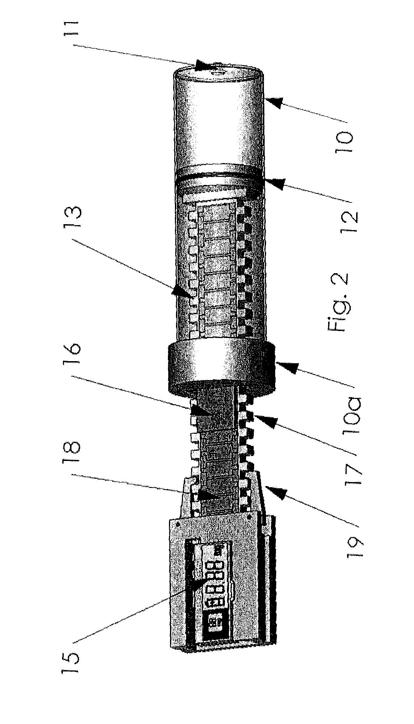

- la figure 2 montre ce stylo débarrassé de son fourreau ; et
- la figure 3 est une vue en coupe simplifiée du stylo. Mode(s) de réalisation de l'invention

Par souci de clarté, les éléments communs aux trois figures ont été désignés par les mêmes références.

Le stylo injecteur selon l'invention est une seringue qui comporte principalement :
- un corps de pompe cylindrique 10 muni, à son extrémité aval, d'une aiguille 11 et destiné à contenir le liquide à injecter soit directement soit dans une cartouche pré remplie;
- un piston 12 capable de coulisser dans le corps de pompe 10;
- une tige 13 à l'extrémité aval de laquelle est fixé le piston 12;
- un fourreau cylindrique 14 solidaire de la tige 13 et dimensionné de manière à coulisser autour du corps de pompe 10;
- un premier boîtier électronique 15 disposé solidairement à l'intérieur du fourreau 14 et, comme précisé plus loin, capable d'être déplacé le long de la tige 13; et
- un deuxième boîtier électronique 16 solidaire du couvercle amont 10a du corps de pompe.

La tige 13 se présente sous la forme d'une règle à section rectangulaire dotée latéralement de deux rangées de crans 17 et formée d'une succession de bobines inductives 18, comme décrit en détail dans le document EP 0785145.

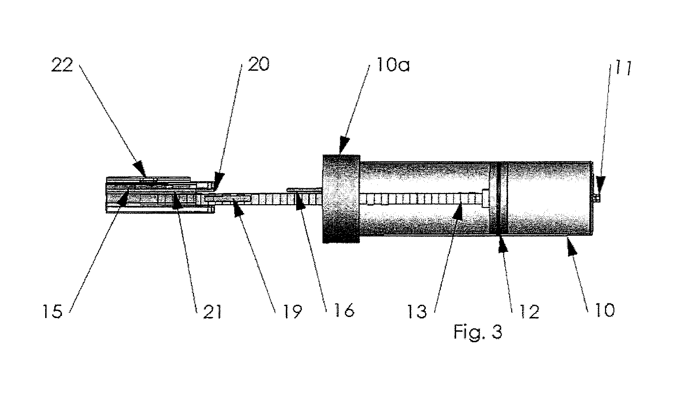

Le boîtier électronique 15, monté coulissant sur la tige 13, est doté de deux lames mobiles 19 coopérant avec les crans 17 et venant en butée sur le couvercle 10a du corps de pompe 10. La figure 3 montre que ce boîtier contient un capteur 20, une électronique 21 recevant et traitant les informations fournies par le capteur 20 et un affichage 22 des indications délivrées par l'électronique 21. La fonction des composants contenus dans le boîtier 15 ne sera pas décrite ici en détail car elle est celle exposée dans le document EP 0 785 415 déjà cité. On se contentera de mentionner que l'électronique 21 détermine, en se basant sur les lois physiques de l'induction, la course du boîtier 15 par rapport à la tige 13 qui reste fixe. II s'agit donc d'une mesure inductive linéaire. La course ainsi déterminée définit la quantité de liquide à injecter. L'affichage 22 fournit en clair une valeur de cette quantité.

Le boîtier 16, monté de manière fixe sur le couvercle 10a du corps de pompe 10, contient un capteur avantageusement identique au capteur 20 du boîtier 15 et est connecté à l'électronique 21 de ce dernier. Par une mesure inductive linéaire, selon les principes déjà mentionnés, l'électronique 21 détermine le déplacement de la tige 13 du piston 12 par rapport au boitier 16 et l'affichage 22 fournit en clair une valeur de la quantité de liquide qui a été effectivement injectée.

En fonctionnement, il s'agit, d'abord, d'aspirer dans la seringue la quantité de liquide désiré ou d'y insérer une cartouche de liquide pré remplie. Dans le premier cas, l'aiguille 11 est plongée dans un réservoir contenant ce liquide et le fourreau 14 est tiré vers l'amont. Lors de ce mouvement, l'affichage 22 fournit au patient une indication de la quantité de médicament aspiré. La dose de liquide à injecter est ainsi déterminée par la position du boitier électronique 15 sur la tige du piston 13.

Lorsque le patient veut, ensuite, s'injecter cette dose, il pousse vers l'aval le fourreau 14 jusqu'à ce que le boîtier 15, solidairement de la tige 13, vienne buter sur le couvercle 10a. Lors de ce mouvement, l'affichage 22 fournit au patient une indication de la quantité de liquide effectivement injectée, vérifiant ainsi qu'elle correspond à l'indication, initialement fournie, de la quantité de liquide prévue. II est à noter qu'il n'est pas nécessaire que le boitier 15 revienne en butée sur le corps de pompe 10. L'affichage 22 indique alors au patient la quantité effective de dose injectée, cette quantité ne correspondant pas à l'intégralité de la quantité de médicament aspiré et l'informe éventuellement de la quantité de liquide restant à disposition dans le corps de la seringue 10.

Ainsi est proposée une seringue facile à utiliser, sans nécessiter aucun équipement extérieur et fournissant au patient les indications de la dose à injecter, de la dose réellement injectée et de la dose restante dans la seringue.

Bien entendu, la présente invention est susceptible de diverses variantes et modifications qui apparaîtront à l'homme de l'art. En particulier, l'affichage peut être associé, à l'usage de patients mal-voyants, à un microphone diffusant les valeurs affichées. Par ailleurs, le stylo peut être doté de moyens aptes à communiquer les données mesurées à une unité de contrôle externe appartenant au patient, à un médecin ou à une clinique par des moyens de communication occupant une place réduite.

## Revendications

1. Stylo injecteur de médicament liquide, **caractérisé en ce qu'**il est constitué d'une seringue comportant :
- un corps de pompe (10) destiné à contenir le médicament à injecter et muni d'une aiguille (11) ou d'une cartouche de liquide pré remplie,
- un piston (12) capable de coulisser dans le corps de pompe (10),
- une tige (13) à une extrémité de laquelle est fixé le piston (12),
- un fourreau (14) solidaire de la tige (13), capable de coulisser autour du corps de pompe (10),
- un boîtier (15) disposé à l'intérieur du fourreau (14) et capable d'être positionné en divers endroits de la tige (13) de manière à ajuster la course du piston (12),
- des moyens de mesure inductive (18, 20) de la course à effectuer par le piston (12),
- des moyens de détermination (21) de la quantité de médicament injectable qui correspond à la course mesurée du piston (12), et
- des moyens d'affichage (22) de ladite quantité.

2. Stylo injecteur selon la revendication 1, **caractérisé en ce que** lesdits moyens de mesure (18, 20) sont des moyens de mesure inductive linéaire.

3. Stylo injecteur selon la revendication 2, **caractérisé en ce que** la tige (13) est sous le forme d'une règle dotée de crans (17) et **en ce que** ledit boîtier (15) est doté de lames mobiles (19) coopérant avec lesdits crans (17).

4. Stylo injecteur selon l'une des revendications 2 et 3, **caractérisé en ce que** les moyens de mesure (18, 20) de la course à effectuer par le piston (12) comprennent une succession de bobines inductives (18) disposées tout au long de la tige (13) et, à l'intérieur du boîtier (15), un capteur (20) coopérant avec lesdites bobines (18).

5. Stylo injecteur selon l'une des revendications 1 à 4, **caractérisé en ce que** lesdits moyens de détermination (21) de la quantité de médicament injectable et les moyens d'affichage (22) sont disposés à l'intérieur du boitier (15).

6. Stylo injecteur selon l'une des revendications 1 à 5, **caractérisé en ce que** la seringue comporte, en outre :
- un capteur monté de manière fixe sur le corps de pompe (10) et coopérant avec lesdites bobines (18) pour fournir une mesure inductive linéaire du déplacement effectif du piston (12), et
- des moyens de détermination (21) de la quantité de médicament injectée qui correspond au déplacement mesuré du piston (12), et
- des moyens d'affichage (22) de ladite quantité.

7. Stylo injecteur selon la revendication 6, **caractérisé en ce que** les moyens de détermination (21) de la quantité de médicament injecté et les moyens d'affichage (22) de ladite quantité sont disposés à l'intérieur dudit boitier (15).
